# EUROPEAN PATENT SPECIFICATION

(11) **EP 2 777 617 B1**
(45) Date of publication and mention of the grant of the patent: **14.09.2022**
(21) Application number: 14159378.0
(22) Date of filing: 13.03.2014
(51) Int. Cl.: A61F 2/24

(54) **Prosthesis with outer skirt**
Prothese mit äußerem Rand
Prothèse avec jupe externe

(30) Priority: 15.03.2013 US 201361798115 P; 15.03.2013 US 201361789783 P
(43) Date of publication of application: 17.09.2014
(73) Proprietor: Edwards Lifesciences CardiAQ LLC, Irvine, CA 92614 (US)
(72) Inventor: Ratz, J. Brent, Irvine, California 92618 (US); Quadri, Arshad, Irvine, California 92618 (US); Pesce, Luca, Irvine, California 92618 (US)
(74) Representative: Fish & Richardson P.C.

(56) References cited:
- WO-A2-2013/028387
- WO-A2-2014/110171
- US-A1- 2006 058 872
- US-A1- 2008 221 672
- US-A1- 2011 137 397
- US-A1- 2011 224 785

## Description

### BACKGROUND OF THE INVENTION

### Field of the Invention

The present disclosure relates generally to prostheses for implantation within a lumen or body cavity. In particular, certain embodiments relate to expandable prostheses such as replacement heart valves, such as for the mitral valve, that are configured to atraumatically grasp intralumenal tissue.

### Description of the Related Art

Human heart valves, which include the aortic, pulmonary, mitral and tricuspid valves, function essentially as one-way valves operating in synchronization with the pumping heart. The valves allow blood to flow downstream, but block blood from flowing upstream. Diseased heart valves exhibit impairments such as narrowing of the valve or regurgitation, which inhibit the valves' ability to control blood flow. Such impairments reduce the heart's blood-pumping efficiency and can be a debilitating and life threatening condition. For example, valve insufficiency can lead to conditions such as heart hypertrophy and dilation of the ventricle. Thus, extensive efforts have been made to develop methods and apparatuses to repair or replace impaired heart vaives.

Prostheses exist to correct problems associated with impaired heart valves. For example, mechanical and tissue-based heart valve prostheses can be used to replace impaired native heart valves. More recently, substantial effort has been dedicated to developing replacement heart valves, particularly tissue-based replacement heart valves that can be delivered with less trauma to the patient than through open heart surgery. Replacement valves are being designed to be delivered through minimally invasive procedures and even percutaneous procedures. Such replacement valves often include a tissue-based valve body that is connected to an expandable frame that is then delivered to the native valve's annulus.

Development of prostheses including but not limited to replacement heart valves that can be compacted for delivery and then controllably expanded for controlled placement has proven to be particularly challenging. An additional challenge relates to the ability of such prostheses to be secured relative to intralumenal tissue, e.g., tissue within any body lumen or cavity, in an atraumatic manner. Further challenges arise when trying to controllably deliver and secure such prostheses in a location such as at a native mitral valve. These replacement valves are often intended to at least partially block blood flow. However, a problem occurs when blood flows around the valve on the outside of the prosthesis. For example, in the context of replacement heart valves, paravalvular leakage has proven particularly challenging.

US 2011/0137397 A1 discloses a prosthesis in accordance with the preamble of claim 1.

US 2011/0224785 A1 teaches a prosthetic valve comprising a collapsible flexible support frame, which is at least partially covered by a covering.

US 2008/0221672 A1 discloses a heart valve prosthesis that includes a flexible ring, wherein a plurality of leaflet membranes, a skirt, and a resilient element are mounted with respect to the flexible ring.

US 2006/0058872 A1 discloses a seal which covers proximal and distal grasping elements and extends between the grasping elements. The seal forms a tubular seal structure exterior to the body or braid of anchor which can conform and seal against paravalvular leaks.

WO 2013/028387 A2 discloses a flexible member covering a stent and spanning between the stent and a support structure.

WO 2014/110171 A2 forms part of the prior art under Art. 54(3) EPC and discloses a valve prosthesis including a stent and an anti-paravalvular leakage component with a skirt formed of a flexible material and a radially expandable control ring that extends a skirt edge outwardly away from the outer surface of the tubular stent and against the native heart valve to form an open-ended annular pocket between the skirt and the outer surface of the tubular stent.

### SUMMARY OF THE INVENTION

Embodiments of the present disclosure are directed to a prosthesis, such as but not limited to a replacement heart valve. The invention is defined by the appended claims and relates to a prosthesis to grasp intraluminal tissue when deployed within a body cavity.

Insofar as the term "embodiment" is used in the following disclosure and/or features are presented as being optional, this should be interpreted in such a way that the only protection sought is that of the invention as claimed.

According to the invention, the prosthesis is configured to be deployed within a body cavity and prevent axial flow of fluid around an exterior of the prosthesis. The prosthesis includes an expandable frame configured to radially expand and contract for deployment within the body cavity, and an outer skirt positioned annularly around an exterior of the expandable frame. In some embodiments, the outer skirt can extend outward from the frame and be secured to an outwardly extending anchor on the frame to create an axial barrier to fluid flow exterior to the frame when deployed within the body cavity.

The prosthesis is configured to grasp intralumenal tissue when deployed within a body cavity and prevent axial flow of fluid around an exterior of the prosthesis. The prosthesis comprises an expandable frame and a plurality of anchors (for example a plurality of proximal anchors and/or a plurality of distal anchors), and a skirt. The expandable frame comprises a proximal end and a distal end and a longitudinal axis extending therethrough, the frame configured to radially expand and contract for deployment within the body cavity. The plurality of proximal anchors each connect to the frame so that when the frame is in an expanded configuration an end of each proximal anchor is positioned radially outward from the frame and extends generally distally. If provided, the plurality of distal anchors can each connect to the frame so that when the frame is in an expanded configuration an end of each distal anchor is positioned radially outward from the frame and extends generally proximally. When both proximal and distal anchors are provided, the ends of the distal anchors are axially spaced from the ends of the proximal anchors when the frame is in an expanded configuration. The skirt is annularly positioned around an exterior of the expandable frame. In some embodiments, the skirt is secured to the tips of the proximal anchors, to create an axial barrier to fluid flow exterior to the frame when deployed within the body cavity. In some embodiments, the frame can be configured such that radial expansion of the frame causes the ends of the plurality of proximal anchors and the ends of the plurality of distal anchors to draw closer together.

In some embodiments a prosthesis can comprise an expandable frame, a plurality of distal anchors and a plurality of proximal anchors. The anchors can extend outwardly from the frame. The frame is configured to radially expand and contract for deployment within the body cavity. When the frame is in an expanded configuration, the proximal anchors can extend a significant distance away from the exterior of the frame, such as a length of about one-half or more the diameter of the frame. In some embodiments, at least some of the anchors comprise a loop that forms an atraumatic end of a corresponding anchor. The skirt is positioned annularly around an exterior of the expandable frame and is connected to the tips of the proximal anchors to create an axial barrier to fluid flow exterior to the frame when deployed within the body cavity.

In some embodiments, a prosthesis comprises an expandable frame, a plurality of proximal anchors connected to the frame, and a plurality of distal anchors connected to the frame. The expandable frame comprises a proximal end and a distal end and a longitudinal axis extending therethrough, the frame configured to collapse radially for delivery and to expand radially upon deployment. The plurality of proximal anchors are expandable to a configuration wherein a portion of each of the proximal anchors extends generally distally and an end of each of the proximal anchors is positioned radially outward from the frame. The plurality of distal anchors can be expandable to a configuration wherein a portion of each of the distal anchors extends generally proximally and an end of each of the distal anchors is positioned radially outward from the frame. Expansion of the frame from a first at least partially collapsed size to a second expanded size can cause the ends of the proximal anchors and the ends of the distal anchors to draw closer together. At least some of the plurality of proximal anchors can be configured to expand to a radial distance from a central longitudinal axis of the frame that is about 150% or more of a radius of the frame when the frame is in an expanded configuration.

### BRIEF DESCRIPTION OF THE DRAWINGS

These and other features, aspects and advantages are described below with reference to the drawings, which are intended to illustrate but not to limit the invention. In the drawings, like reference characters denote corresponding features consistently throughout similar embodiments.
Figure 1A is a side view of a frame for a prosthesis.
Figure 1B is a top perspective view of the frame of Figure 1A where the anchors have not yet been bent to the desired shape.
Figure 2 is an embodiment of a prosthesis configured as a replacement heart valve.
Figure 3 is an embodiment of a prosthesis configured as a replacement heart valve.
Figure 4 is an embodiment of a prosthesis configured as a replacement heart valve.
Figure 5 is a schematic representation of a prosthesis positioned within the heart.
Figure 6 is a detailed schematic representation of the prosthesis positioned within the heart.
Figure 7A is a top perspective view of an embodiment of a prosthesis configured as a replacement heart valve.
Figure 7B is a bottom perspective view of the prosthesis of Figure 7A.
Figure 7C is a side view of the prosthesis of Figure 7A.
Figure 7D is a side view of the frame from the prosthesis of Figure 7A.
Figure 8 is a perspective view of a skirt.
Figure 9A is a schematic representation of a prosthesis positioned within the heart.
Figure 9B is a detailed schematic representation of the prosthesis positioned within the heart of Figure 9A.

### DETAILED DESCRIPTION OF THE PREFERRED EMBODIMENTS

The present specification and drawings provide aspects and features of the disclosure in the context of several embodiments of prostheses, replacement heart valves, delivery devices and methods that are configured for use in the vasculature of a patient, such as for replacement of natural heart valves in a patient. These embodiments may be discussed in connection with replacing specific valves such as the patient's aortic or mitral valve. However, it is to be understood that the features and concepts discussed herein can be applied to products other than heart valve implants. For example, the controlled positioning, deployment, and securing features described herein can be applied to medical implants, for example other types of expandable prostheses, for use elsewhere in the body, such as within a vein, or the like. In addition, particular features of a valve, delivery device, etc. should not be taken as limiting, and features of any one embodiment discussed herein can be combined with features of other embodiments as desired and when appropriate.

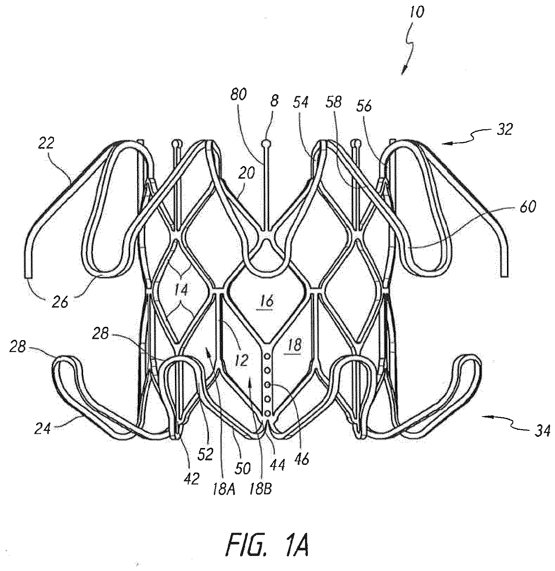

With initial reference to Figures 1A-B, an embodiment of a prosthesis 10 is shown. The illustrated prosthesis 10 includes a frame 20 that may be self-expanding or balloon expandable. The frame 20 can include a proximal end 32, a distal end 34 and proximal 22 and distal 24 anchors. The anchors can allow the frame to engage a native valve annulus or other tissue to be implanted at a target location. The prosthesis 10 can include one or more of a valve 60, an outer skirt 30, and a valve skirt 70 as will be described in more detail below. The valve 60 can be designed to replace a damaged or diseased native heart valve such as a mitral valve; though it will be understood that a replacement valve is not required as part of the prosthesis.

The prosthesis can be a replacement heart valve similar to that and including features similar to those disclosed in U.S. Provisional Appl. Nos. 61/782,707, filed March 14, 2013 and 61/789,783 filed March 15, 2013, U.S. Patent No. 8,403,983 and U.S. Publication Nos. 2010/0298931, 2011/0313515 and 2012/0078353.

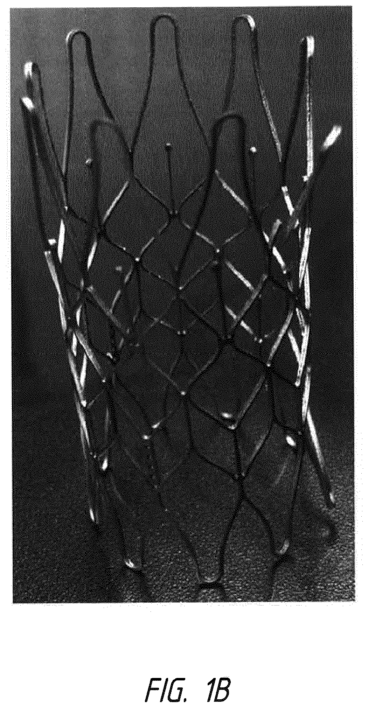

The frame 20 can be made of many different materials, but is preferably made from metal. In some embodiments, the frame 20 can be made from a shape memory material, such as nitinol. A wire frame or a metal tube can be used to make the frame. The wire frame of a metal tube can be cut or etched to remove all but the desired metal skeleton. In some embodiments a metal tube is laser cut in a repeating pattern to form the frame. The flat pattern can be cut from a metal tube and then the tube can be bent and expanded to the shape shown in Figures 1A-B. The frame 20 can further be expanded and/or compressed and/or otherwise worked to have the desired shape or shapes, such as for introduction and implantation. Figure 1B shows the frame 20 in a partially finished state. The frame has been cut and initially expanded, but the anchors 22, 24 have not yet been bent as shown in Figure 1A.

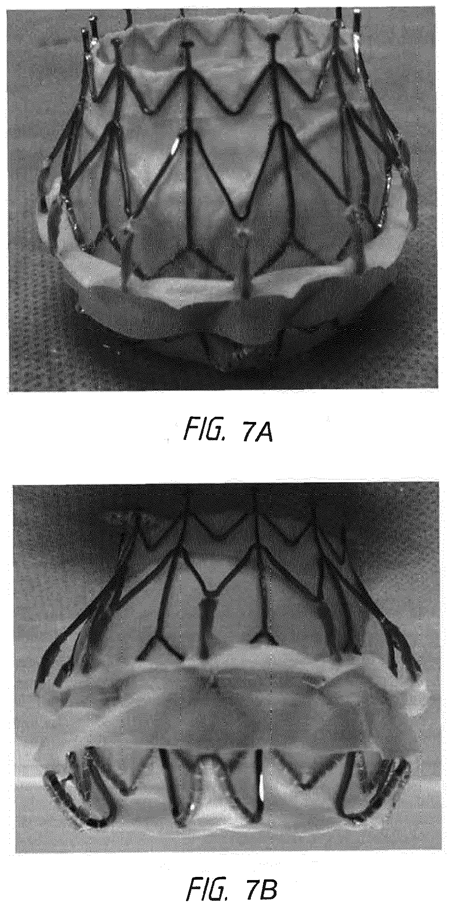

As shown, the frame when in an expanded configuration, such as in a fully expanded configuration, has a cylindrical or slightly cylindrical shape, where a middle portion is substantially similar in shape and size as the proximal 32 and distal 34 ends. The frame can be a substantially cylindrical shape with the same or substantially the constant cross-sectional dimension or diameter from the proximal end to the distal end. The cylindrical shape of the frame, in combination with the anchors described below, can advantageously allow the frame to float within a native valve while the anchors engage a native valve annulus or other body cavity and spacing the inlet and outlet of the frame away from the heart or vessel wall. This can help reduce undesired contact between the prosthesis and the heart or vessel, such as the ventricular wall of the heart. The prosthesis 10 and frame 20 may be similar to the replacement heart valves and associated frames disclosed in U.S. Provisional Appl. Nos. 61/782,707, filed March 14, 2013 and 61/789,783 filed March 15, 2013, U.S. Patent No. 8,403,983 and U.S. Publication Nos. 2010/0298931 and 2011/0313515. Further examples of frame and prosthesis configurations that may also be utilized are described with respect to Figures 7A-9B.

A number of struts collectively make up the frame 20. Figure 1A illustrates the frame in an expanded configuration with a number of longitudinal struts 12 and undulating struts 14, with cells 16, 18 defined by the open spaces between the struts. The longitudinal struts may be arranged so that they are parallel or generally or substantially parallel to a longitudinal axis of the frame. The longitudinal axis of the frame may be defined as the central axis that extends through the center of the frame between the proximal 32 and distal 34 ends. Any number of configurations of struts can be used, such as the rings of undulating struts shown forming chevrons and diamonds, but also ovals, curves, and various other shapes The illustrated embodiment includes two rows of diamond-shaped cells 16 at the top or adjacent the proximal end and then a row of sideways diamond-shaped cells 18 at the bottom or near the distal end. These sideways diamond-shapes are made of two longitudinal struts 12 that are offset from one another, or one is higher than the other, and then an undulating strut 14 connects the longitudinal struts 12 at the top and bottom of the cell. The sideways diamond-shapes alternate between sides as to which side is higher and which is lower. So a first sideways diamond-shaped cell 18A has a low left side and a high right side, and a second sideways diamond-shaped cell 18B has a high left side and a low right side, where the high right side of cell 18A shares the same longitudinal strut 12 as the high left side of cell 18B.

Some of the struts can include one or more eyelets 46. As illustrated, a plurality of eyelets, here five, are located along one of the longitudinal struts 12 as part of the sideways diamond-shaped cells. One or more eyelets can be positioned in other locations along the frame and/or anchors. The eyelets 46 may be used to attach features such as the valve 60, outer skirt 30, and/or valve skirt 70 to the frame 20.

The frame 20 as illustrated is foreshortening. The foreshortening can be defined by the frame 20 and the positioning of various types of struts along the frame 20. When the frame is radially collapsed or compacted, the struts 14 become more parallel with respect to the longitudinal axis of the frame, causing cells 16 and 18 to collapse, causing an outer diameter of the frame to decrease and the longitudinal length of the frame to increase. As the frame moves from a compacted position to an expanded position, the cells 16 and 18 widen sideways and the longitudinal length of the frame can decrease. It will be appreciated while in some embodiments the entire length of the frame 20 is foreshortening, in other embodiments such as embodiments of the prostheses described in the patents and applications incorporated by reference herein, only a portion of the frame is foreshortening.

Foreshortening of the frame 20 can be used to engage and secure the prosthesis to intralumenal tissue in a body cavity, for example tissue at or adjacent a native valve, such as a native valve annulus and/or leaflets. Opposing anchors 22, 24 can be constructed on the frame 20 so that portions of the anchors, such as tips or ends 26, 28, move closer together as the frame foreshortens. As one example, this can allow the anchors 22, 24 to grasp tissue on opposite sides of the native mitral annulus to thereby secure the prosthesis at the mitral valve.

The anchors 22, 24 and anchor tips 26, 28 can be located anywhere along the frame 20 just so long as at least one of the anchors is either connected to a foreshortening portion or a foreshortening portion is positioned between the anchors so that a portion of the anchors will be move closer together with expansion of the frame. As shown in Figure 1A, the foreshortening portion extends the entire or substantially the entire length of the frame.

Preferably, each of the anchors 22, 24 is positioned or extends generally radially outwardly from the frame 20 so that the anchor tips 26, 28 are generally spaced away or radially outward from the rest of the frame 20. For example, the anchor tips may be located radially outward from the middle portion of the frame, with the tips 26 and 28 being axially spaced from one another. In some embodiments, all or part of the structure connected to the anchor tip and extending radially from the frame, including one or more rings and/or struts, can be considered part of the anchor. The anchors can include a base located on the anchor on a side opposite the tip. The base can be for example where the anchor begins to extend from or away from the frame 20.

For example, the distal anchors 24 are shown having looped anchors. Each looped anchor has a first base 42 and a second base 44 connected to the frame, wherein the first and second bases are at the distal-most corners of adjacent cells. Alternatively, the first and second bases may be located at adjacent corners of the same cell, or at opposite corners of the same cell if for example the cells adjacent the distal end of the frame have the same configuration as the cells shown in Figure 1A at the proximal end of the frame. The distal anchors 24 extend generally distally away from the frame and are bent near the base 42 to extend radially outward away from the frame along a first segment 50. Then the anchor is bent to point proximally or generally proximally along a second segment 52 ending in the tip 28. The tips 28 of the distal anchors may be curved or arcuate atraumatic tips. The ends of the distal anchors can extend proximally and be parallel or substantially parallel with the longitudinal axis of the frame, or they may extend generally proximally but still radially outwardly inclined or at an acute angle relative to the longitudinal axis of the frame. The distal anchor 24 then repeats this configuration in reverse towards the second base 44 such that the two sides of the looped anchor are mirror images of one another.

In addition, as illustrated in Figure 1A, adjacent distal anchors share one strut so that each distal anchor is directly adjacent another distal anchor. In other embodiments, distal anchors can be spaced apart, for example with at least a cell 18 or two cells 18 located between the distal anchors. It will be understood that the distal anchors can have other configurations such as described in the applications included in the applications incorporated by reference, and for example as shown in Figures 7A-9B, and that the distal anchors may not be symmetrical.

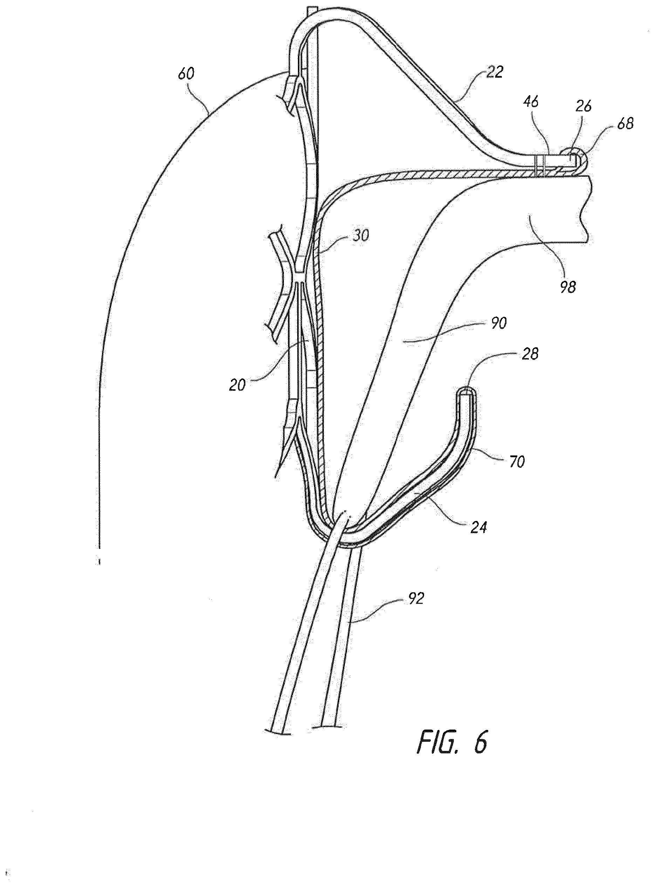

The proximal anchors 22 are shown having looped anchors of a similar shape and configuration as the distal anchors 24. It can be seen that each proximal anchor extends from or near the proximal end of the frame at bases 54 and 56 located on adjacent proximal-most corners of cells 16. In the embodiment illustrated in Figure 1A, the proximal anchors are longer than the distal anchors, and as such, the proximal anchors may extend further away from the frame. As shown the anchors extend proximally at the bases 54, 56 and are then bent to segment 58 which extends radially outwardly away from the frame in a generally distal direction. Then the proximal anchor is bent to point distally or generally distally along a second segment 60 ending in the tip 26. The tips 26 of the proximal anchors may be curved or arcuate atraumatic tips. The ends of the proximal anchors can extend distally and be parallel or substantially parallel with the longitudinal axis of the frame, or they may extend generally distally but still radially outwardly inclined or at an acute angle relative to the longitudinal axis of the frame. Another embodiment, described below with respect to Figure 6, has the ends of the proximal anchors becoming perpendicular to the longitudinal axis of the frame.

In addition, adjacent proximal anchors can share the same base where the anchors are bent outward from the frame, or may be considered to extend from the same corner on cell 16. In other embodiments, proximal anchors can be spaced apart so that there is at least a ½ cell or one cell located between the proximal anchors. It will be understood that the proximal anchors can have other configurations such as described in the applications incorporated by reference and for example, as shown in Figures 7A-9B, and that the proximal anchors may not be symmetrical. As illustrated in Figure 1A, between the bases 54 and 56 of each proximal anchor, there may also be located a longitudinally extending strut 80 extending proximally, e.g. from the proximal-most corner of a cell 16 in the second row of cells from the proximal end. These struts 80 may terminate at their proximal ends in an enlarged portion such as a tab 8 that may facilitate holding or retaining the proximal end of the frame, such as in a delivery system as described below.

In some embodiments, in an expanded state such as shown in Figure 1A, at least some of the proximal anchors can extend to a radial distance from an exterior surface of the frame that is ½ (or about ½) or more of the expanded diameter of the frame. In some embodiments, all of the proximal anchors extend at least to this radial distance. In even further embodiments, all of the proximal and distal anchors extend at least to this radial distance. In other embodiments, the radial distance of one or more of the ends of the anchors from a central longitudinal axis passing through the middle of the frame may be 150% (or about 150%) or more, 180% (or about 180%) or more, 200% (or about 200%) or more, 220% (or about 220%) or more, or 250% (or about 250%) or more of the radius of the frame when the frame and the anchors are in expanded configurations. For example, if the radius of the frame is 16 mm and a proximal anchor end is spaced 9 mm from the exterior of the frame, that proximal anchor extends 25 mm from the central longitudinal axis of the frame, and is 156.25% of the radius of the frame.

In some embodiments the diameter of the frame 20 may be in the range of 20-40 mm (or about 20 to about 40 mm), more preferably 25-35 mm (or about 25 to about 35 mm) when expanded. The outermost tip diameter may be greater than the frame diameter as described above and may be in the range of 40-60 mm (or about 40 to about 60 mm), and in some embodiments may be about 50 mm when the frame diameter is about 30 mm. In some embodiments the length of the prosthesis, from proximal to distal end, when compressed, is between 20-40 mm (or about 20 to about 40 mm), more preferably 25 to 30 mm (or about 25 to about 30 mm), for example about 29 mm. When expanded, the prosthesis may have a length between 15 to 20 mm (or about 15 to 20 mm), more preferably 17 to 18 mm (or about 17 to about 18 mm).

The distal anchors 24 can be positioned to be not as far radially outward as the proximal anchors, and the tips 28 may be positioned radially inward of the tips 26. As described further below, such a configuration may be advantageous in positioning and securing the prosthesis in a mitral valve or other body location. In some embodiments, as illustrated in Figure 1A, the ends or tips 26 of the proximal anchors 22 are positioned further radially outward from the frame 20 than the ends of tips 28 distal anchors 24 when the frame and the anchors are in an expanded configuration, e.g., when they are fully expanded. In other embodiments, the distal anchors and proximal anchors can be positioned at the same radial outward dimension, or the distal anchors may even be positioned further outward than the proximal anchors. In further embodiments, some of the proximal anchors (or distal anchors) may extend to a first radial distance, and others of the proximal anchors (or distal anchors) may extend to a second radial distance, where the first radial distance is greater than the second radial distance. The distal anchors in Figure 1A are shown to be circumferentially staggered with respect to the proximal anchors, meaning that the tips 26 of the proximal anchors are not aligned, and are circumferentially in between the tips 28 of the distal anchors. In other embodiments, the tips 26 and 28 may be circumferentially aligned.

It will be understood that the anchors can have various other configurations. In some embodiments, each of the anchors can extend radially outwardly from the frame at an anchor base and terminate at an anchor tip. The anchors can be connected to the frame at one of many different locations including apices, junctions, other parts of struts, etc. The anchors can comprise first, second, third, or more spaced apart bending stages along the length of each anchor. The anchors can also extend either distally or proximally before and/or after one or more of the bending stages. A portion of the anchor may extend with the frame before or after any bending stages. As shown, the anchors 22, 24 may comprise loops as described above, having a curved or arcuate atraumatic tip to minimize damage to body tissue. Further details that may be incorporated and/or interchanged with the features described herein are disclosed in U.S. Provisional Appl. Nos. 61/782,707, filed March 14, 2013 and 61/789,783 filed March 15, 2013, U.S. Patent No. 8,403,983 and U.S. Publication Nos. 2010/0298931, 2011/0313515 and 2012/0078353. For example, the frame 20 and/or the anchors 22, 24 may have configurations as shown with respect to Figures 7A-9B.

As shown in Figure 1B, in one embodiment the prosthesis has nine distal anchors and nine proximal anchors. Any number of proximal and distal anchors may be used. In other embodiments, instead of a 1:1 correspondence between anchors, other ratios, such as a 9:6 or a 9:3 correspondence between the anchors, are possible.

With respect to the number of cells and rows of cells, when there are nine proximal anchors and nine distal anchors, there may be two rows of nine cells 16 each. The cells in the second row can share struts 14 from the first row. A third row at the distal end can have a different cell configuration with sideways diamonds as illustrated in Figure 1A, and can for example have eighteen cells 18, with each cell 18 sharing a strut from a cell in the second row. In other embodiments, the third row can include cells similar in shape to cells 16 in the first and second rows. In another embodiment, a frame may have one or more rows of diamond-shaped cells, where the number of cells per row is 12 or some other number.

The anchor tips 26 and 28 as described above advantageously provide atraumatic surfaces that may be used to grasp intralumenal tissue without causing unnecessary or undesired trauma to tissue. For example, the proximal anchors tips 26 and distal anchor tips 28 may form flat, substantially flat, curved or other non-sharp surfaces to allow the tips to engage and/or grasp tissue, without necessarily piercing or puncturing through tissue. A looped end or looped anchor may assist the frame in not getting caught up on structures at or near the treatment location. For example, each loop can be configured so that when the frame is deployed in-situ and the anchors expand away from the frame, the movement of each loop from a delivered position to a deployed position avoids getting caught on the papillary muscles.

The prosthesis 10 may include a valve 60 as can be seen in schematically in Figure 6. The valve 60 can be a replacement heart valve which includes a plurality of valve leaflets. The plurality of valve leaflets can function in a manner similar to the natural mitral valve, or to other valves in the vascular system. The plurality of valve leaflets can open in a first position and then engage one another to close the valve in a second position. The plurality of valve leaflets can be made to function as a one way valve such that flow in one direction opens the valve and flow in a second direction opposite the first direction closes the valve. The replacement heart valve 60 can be constructed so as to open naturally with the beating of the heart. For example, the plurality of valve leaflets can open during diastole and close during systole.

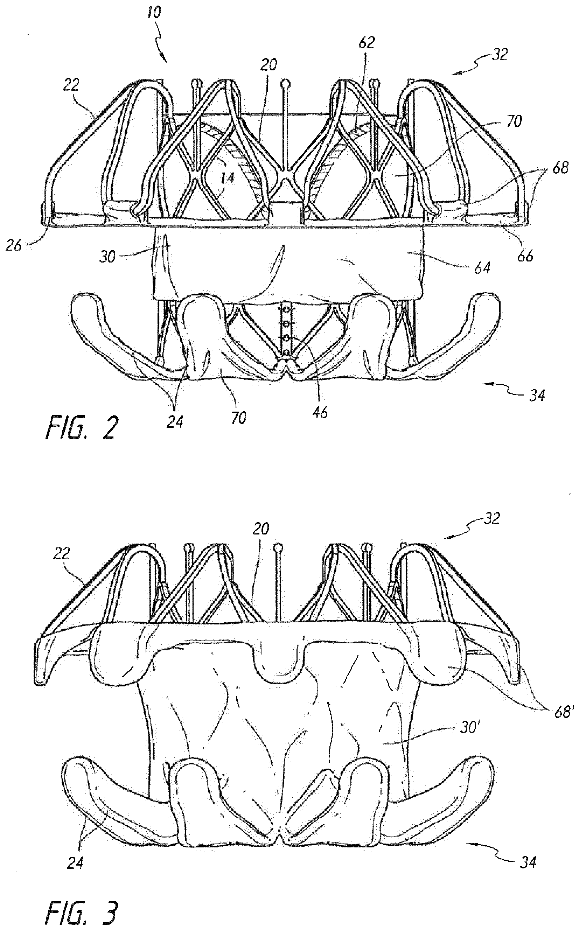

In some embodiments, the leaflets can be coupled to a valve skirt 70. For example, Figure 2 shows a seam 62 where the proximal ends of the leaflets can be connected to the valve skirt 70.

The valve skirt 70 can be used to at least partially control how fluid flows through and/or around the valve 60. The valve skirt 70 can surround at least a portion of the valve and be connected to the valve leaflets. In some embodiments, the valve skirt 70 can form an inner wall connected to and positioned within the frame 20. For example the skirt 70 can connect to the frame at the eyelets 46, such as by stitching. The skirt may also be attached directing to the struts, typically also by stitching. The valve skirt 70 can also be made to move with the foreshortening portion of the frame 20.

The valve skirt 70 can extend the length of the frame 20 or it can extend along only part of the length of the frame 20. In some embodiments, the ends of the heart valve 60 can coincide with ends of the valve skirt 70. In addition, one or more of the ends of the frame 20 can coincide with the ends of the valve skirt 70. In the illustrated embodiment of Figure 2, the proximal end of the valve skirt 70 is positioned proximally from the proximal end of the heart valve 60 as indicated by the seam 62. The valve skirt 70 can not only extend to the distal end of the frame 20 but can also extend to the outside of the frame and is shown attached to and extending to the tip 28 of each distal anchor 24. As shown, the skirt 70 is sewn to each distal anchor.

Other shapes and configurations can also be used for the valve 60 and valve skirt 70. In some embodiments, the valve skirt 70 may extend along the length of the leaflets, but is not connected to them. In the illustrated embodiments, the valve skirt 70 is attached to the frame 20 and the leaflets are attached to the valve skirt 70, such as at the seam 62 in Figure 2.

The valve skirt 70 can be constructed in multiple different ways. The valve skirt 70 can be made a layer of resilient material, such as knit polyester or another stretchable or flexible fabric. In some embodiments, the valve skirt 70 is made from a material that is more flexible than the valve leaflet material. The distal and/or proximal end of the skirt 70 can be straight, curved, or have any other desired configuration. For example, the valve skirt 70 is shown with a straight proximal end at the proximal end 32 of the frame. In other embodiments the skirt distal end can be patterned to generally correspond to the undulations at one end of the frame 20.The valve skirt 70 according to the invention is formed of one piece and can be formed of multiple pieces not according to the invention. For example, the valve skirt 70 attached to the valve 60 can be one piece and then each distal anchor can be covered by a separate piece of material of the valve skirt 70. It is to be understood that other configurations of the valve skirt 70 can also be employed. For example, the anchors may remain uncovered, or only a portion may be covered.

In another embodiment of the valve skirt 70, the end can extend past the frame and can be wrapped around it. Thus, the valve skirt 70 can extend from the inside of the frame 20 to the outside of the frame. The skirt can extend completely around the frame for 1/4, 1/3, 1/2, or more of the length of the distal anchors. Such an embodiment is shown and described with respect to Figures 7A-7B of Provisional Appl. No. 61/782,707. The skirt 70 can also cover the distal anchors 24 as is shown in Figures 7A and 7B of Provisional Appl. No. 61/782,707 and in Figure 2 herein. The skirt can be a one piece skirt, but it will be understood that the skirt can be made of multiple pieces.

The valve skirt 70, and particularly portions that cover the distal anchors 24, can beneficially be used to help prevent leakage of blood flow around the heart valve. In addition, the skirt can encourage tissue in-growth between the skirt and the natural tissue. This may further help to prevent leakage of blood flow around the heart valve.

Continuing to look to Figure 2, an outer skirt or apron 30 is shown that according to the invention also forms part of the prosthesis 10. Figure 2 shows the outer skirt 30 attached to the frame 20 at the tips 26 of the proximal anchors. The outer skirt 30 can have a portion shaped to correspond generally with the shape of an outer portion of the frame 20. According to the invention, a first portion 64 of the outer skirt 30 has a cylindrical or generally cylindrical shape with an inner diameter that substantially corresponds in size to, or according to examples not falling under the scope of the claim, may be larger or slightly larger than, an outer diameter of the frame 20. According to the invention, the outer skirt 30 has a second portion 66 with an annular shape that extends away from the first portion 64 to an outer border with a diameter larger than the diameter of the first portion. As illustrated in Figure 2, the second portion 66 is shown flaring outward from the first portion 64 and extending generally perpendicularly from the first portion 64. Thus, the illustrated second portion forms an annular ring comprising a proximal edge and a distal edge, wherein a diameter of the proximal edge is larger than a diameter of the distal edge.

The outer skirt 30 can attach to the frame, and more preferably attach to the anchors, in one of many different ways. The outer skirt 30 can be sewn to the frame and/or valve skirt. The outer skirt 30 can also be wrapped around a portion of the frame and then sewn to itself. In the embodiment illustrated in Figure 2, the second portion 66 is attached to the proximal anchors 22. For example, a plurality of circumferentially spaced tabs 68 extending radially outward from the proximal edge of the second portion 66 can be used to attach the outer skirt 30 to the proximal anchors. The tabs 68 can be wrapped around the tip 26 (e.g., through the loop) of a proximal anchor and connected to the second portion. The tabs 68 themselves may also form sleeves that are configured to surround at least a portion of the proximal anchors. In some embodiments, the proximal anchors 22 can include eyelets that may be used to secure the skirt to the anchor. The tab 68 can be attached to the eyelet 46, for example by stitching.

In one embodiment, the outer skirt 30 is only attached to the frame via the proximal anchors, and the first portion 64 remains unattached to any portion of the frame or any anchors. In another embodiment, the outer skirt is both attached to the proximal anchors and to the middle portion of the frame. As illustrated in Figure 2, the second portion 66 attached to the anchors extends inwardly from the proximal anchors 22. The first portion 64 then extends distally from the second portion 66 and terminates in a distal edge, which may be free or which may attach to the middle portion of the frame 20 or the skirt 70. In other embodiments, the first portion 64 may also be attached to portions of the frame and/or the distal anchors. In some embodiments, the distal edge of the skirt 30 may be spaced radially outward from the frame when the frame is in an expanded configuration. In addition, the distal edge of the skirt 30 may extend to the distal end of the frame, or it may be spaced proximally therefrom as illustrated in Figure 2.

In some embodiments, the outer skirt 30 can attach to the frame at a distal end of the skirt, or at some other location and then curve up and out towards the proximal anchors. Thus, the outer skirt may not have a distinct first portion and second portion. In still other embodiments, the outer skirt may extend along a substantial portion of the frame. Additional examples of outer skirt features that may be incorporated and/or interchanged with the features described herein are found in U.S. Provisional Application No. 61/789,783 filed March 15, 2013.

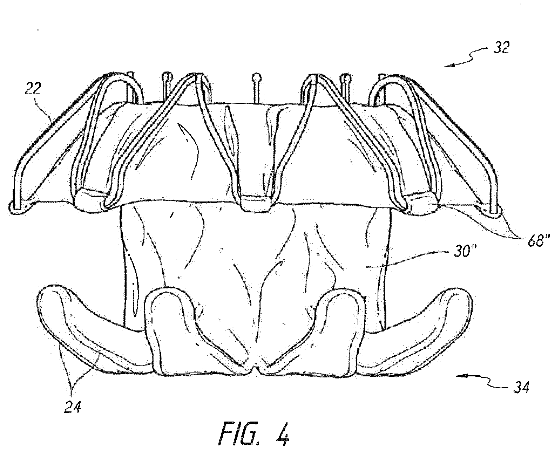

Figures 3 and 4 show additional embodiments of the outer skirt 30', 30". In Figure 3, the outer skirt 30' extends along a substantial part of the frame and extends between proximal and distal anchors. The outer skirt 30' in this embodiment may be one single piece, or may be formed from multiple pieces stitched or otherwise connected together. The tabs 68' may form sleeves that are configured to surround at least a portion of the proximal anchors to attach the outer skirt to the proximal anchors. As shown in one embodiment, proximal portions of the proximal anchors remain uncovered by the outer skirt 30'. The outer skirt 30' at the proximal anchors can form an annular ring similar to the second portion 66 of Figure 2 and can form a substantially cylindrical portion similar to the first portion 64 of Figure 2. The distal end of the outer skirt 30' can attach and/or cover the distal anchors 24. In some embodiments, the distal end of the outer skirt 30' extends to the distal anchors 24 but does not cover or connect to them. In addition, a valve skirt 70 may also be used which may create some overlap of skirts in some embodiments.

In Figure 4 the outer skirt 30" is shown attached to the distal ends of the proximal anchors 22 with tabs 68". The outer skirt 30" then extends proximally to essentially the base of the proximal anchor. From that point, the outer skirt extends distally towards the distal end. Again the outer skirt may or may not attach to the distal anchors. In the illustrated embodiment of Figure 4, the outer skirt 30" wraps around each of the distal anchors.

Turning now to Figure 6, a detail view of an embodiments is shown where the proximal anchors 22 can include eyelets 46 that may be used to secure the skirt to the anchor. In this embodiment, the proximal anchor has an end 26 that instead of extending generally distally, it extends generally radially outwardly, and as illustrated extends in a direction perpendicular or substantially perpendicular to the longitudinal axis of the frame. The tab 68 can be attached to the eyelet 46, for example by stitching. As shown, the eyelet 46 is positioned at the end of the anchor, but it will be understood that it can be spaced proximally from the end. In some embodiments, the proximal anchors can be looped anchors or have a looped end. A small tab can be passed through the looped anchor or looped end and connected to the skirt to form a loop on the skirt. Further, the outer skirt 30 may attach directly to the eyelets 46 without the need for tabs 68.

In some embodiments, the outer skirt 30 can be part of, or connected to, the valve skirt 70, such as being connected to the valve skirt 70 at or near the distal end 34 of the frame.

The outer skirt 30 can be constructed in multiple different ways and may be made of similar material to the valve skirt 70. The outer skirt 30 can be made of a layer of resilient material, such as knit polyester or another stretchable or flexible fabric. In some embodiments, the outer skirt 30 is made from a material that is more flexible than the valve leaflet material. The distal and/or proximal end of the outer skirt 30 can be straight, curved, or have any other desired configuration. The outer skirt 30 can be formed of one piece (according to the invention) or multiple pieces (not according to the invention). For example, the outer skirt 30 attached to the frame 20 can be one piece and then each proximal anchor 22 can be covered by a separate piece of material of the outer skirt 30. It is to be understood that other configurations of the outer skirt 30 can also be employed. For example, the anchors may remain uncovered, or only a portion may be covered.

The outer skirt 30 can beneficially prevent axial flow of fluid around an exterior of the prosthesis. For example, with the outer skirt 30 be positioned annularly around an exterior of the expandable frame and secured to at least some of the plurality of proximal anchors, the outer skirt creates an axial barrier to fluid flow exterior to the frame when deployed within a body cavity. In addition, the skirt can encourage tissue in-growth between the skirt and the natural tissue. This may further help to prevent leakage of blood flow around the heart valve.

In one embodiment, the outer skirt 30 can be used to help prevent leakage of blood flow around a heart valve, such as a mitral valve, when the prosthesis is placed in a native heart valve. For example, the outer skirt 30 can engage an atrial side of the mitral valve. The proximal anchors can also engage the mitral valve forcing the outer skirt 30 into close contact with the valve to block flow from passing through the mitral valve from outside of the frame.

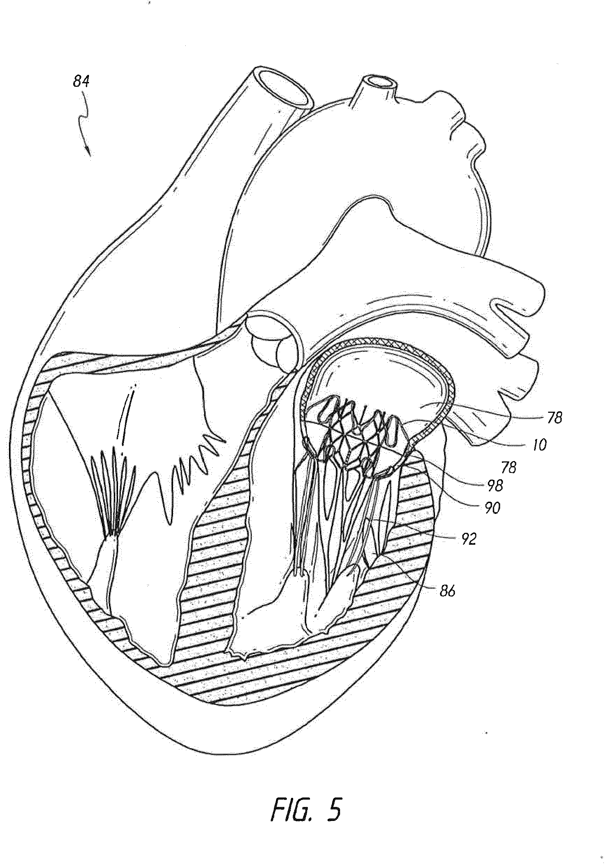

In preferred embodiments, the prostheses 10 in the form of a replacement heart such as described above may be deployed into a heart valve annulus. The prosthesis 10 may be delivered into the mitral valve in a radially compacted or collapsed configuration and positioned when compacted so that the anchor tips 26, 28 of the opposing anchors 22, 24 are disposed on opposite sides of the native annulus 98 as shown in Figures 5 and 6. In Figure 5, the valve 60 and skirts are not shown for ease of illustration. As the replacement heart valve 10 is expanded, the opposing anchors expand outward away from the frame are may be drawn closer together due to foreshortening of the frame. The anchors may grasp tissue on opposite sides of the native annulus 98 and securely hold the replacement heart valve 10 in position. As such, the replacement heart valve 10 can be held securely in position without requiring a substantial radial force against the native annulus. Because the anchor tips are preferably atraumatic, the grasping or engaging of tissue by the prosthesis minimizes damage to the native tissue. The prosthesis can be deployed into a heart valve or otherwise deployed in manners similar to those described with respect to a replacement heart valve in U.S. Publication Nos. 2010/0298931 and 2012/0078353.

Figures 5 and 6 show a schematic representation of the replacement heart valve 10 installed in a human heart 84. The heart is shown in cross-section, and represents typical anatomy, including a left atrium 78 and left ventricle 86. The left atrium 78 and left ventricle 86 communicate with one another through a mitral annulus 98. Also shown schematically is a native anterior mitral leaflet 90 having chordae tendineae 92 that connect a downstream end of the anterior mitral leaflet 90 and to the left ventricle 86. Figure 6 shows an enlarged view of a slightly different prosthesis implanted at the native mitral annulus.

An exemplary method of delivering a replacement valve to a native mitral valve and atraumatically securing the replacement valve relative to the native mitral valve annulus 98 is described as follows. The replacement valve can be mounted on a delivery device and delivered to the native mitral valve annulus while the replacement valve is in a radially compacted state. The replacement valve may be positioned so that the ends or tips of the distal anchors are on a ventricular side of the native leaflets 90 beyond a location where chordae tendineae 92 connect to free ends of the native leaflets. At least a portion of the replacement valve can be released from the delivery device to thereby expand the distal anchors radially outwardly. At this time the distal anchors may extend between at least some of the chordae. The distal anchors (along with the frame) can be moved toward the ventricular side of the native valve annulus with the distal anchors extending between at least some of the chordae tendineae to provide tension on the chordae tendineae. With tension provided on the chordae tendineae, the replacement valve can be further released from the delivery device to thereby expand the proximal anchors radially outwardly. The proximal anchors upon further release of the replacement valve from the delivery device can move into engagement with tissue on an atrial side of the native valve annulus, such as with the atrial side of the native valve annulus.

The method just described may utilize any of the prostheses herein described, including those described in the patent and applications incorporated by reference. The illustrated prosthesis where the ends of the distal anchors are not positioned as far out radially as the ends of the proximal anchors when the frame is expanded can beneficially be used in this method. Thus, the distal anchors may have a suitable length for extending between and providing tension on the chordae tendineae, but need not and may in some embodiments not engage tissue with the tips 28, such as shown in Figure 6. Thus, in some embodiments some or all of the distal anchors remain spaced from tissue on the ventricular side of the native valve annulus after delivery and expansion. The interaction between the distal anchors and the chordae tendineae may therefore be sufficient to secure the distal end of the prosthesis, while the engagement of the proximal anchors with tissue on the atrial side of the native valve annulus will help further secure and orient the prosthesis

As illustrated in Figures 5 and 6, the distal anchors may comprise loops, such as any of the looped structures previously described. The proximal and/or distal anchors may also be covered with a resilient material such as described above for the outer skirt 30 and valve skirt 70 that promotes tissue growth with adjacent body tissue. Such material may also be useful to prevent paravalvular leakage. The atraumatic distal anchors may advantageously prevent snagging of the prosthesis on internal structures, such as the papillary muscles.

When the prosthesis is in an expanded configuration within the native mitral heart valve, the engagement of the proximal anchors 22 with tissue on the atrial side of the native mitral valve causes at least a portion of the outer skirt 30 to also engage the tissue on the atrial side of the native mitral valve. A portion of the outer skirt extends distally from the proximal anchors toward the ventricle. Because the diameter of the outer skirt decreases to a size of close or the same in dimension as the frame, the outer skirt form a barrier to blood flow around the outside or external to the frame. The outer skirt 30 can be forced against the outside of the frame 20 by the native leaflets. Where the native leaflets do not force the outer skirt 30 against the frame, or where the contact is not as strong, the outer skirt 30 is still present to block, or impede blood flow. It will be understood that having multiple contact points between the native valve and the outer skirt can allow the outer skirt to securely cover areas where there are fewer contacts between the two. As described above, the outer skirt may also promote tissue growth with tissue that it contacts.

In addition, due to the preferred outer dimension of the anchors relative to the diameter or radius of the frame in some embodiments, when the frame is radially expanded such that the proximal and/or distal anchors engage tissue at or around the native mitral valve annulus, the frame may move reciprocally in an axial direction relative to the native mitral valve annulus in a constrained floating manner. The one or more of the anchors 22, 24 can be made to flex to provide this reciprocal movement; for example, around the bends between the segments 50, 56 and the base of the anchors. The frame does not exert a significant amount of radial force to the native mitral valve annulus or adjacent tissues, and the frame is primarily secured with the anchors. When in use, the frame may then move relative to the anchor ends as the heart is beating.

The embodiments described in Figures 1-6 above may have further advantages. As illustrated in Figure 1, some embodiments have only 3 rows of cells or less, which makes the prosthesis longitudinally shorter and therefore easier to navigate when collapsed through tortuous pathways (e.g., percutaneously). Because of the dimensions of the anchors relative to the size of the frame, the frame itself may be made relatively smaller, which also helps facilitate a lower profile for the prosthesis helpful for delivery and implantation. Moreover, having a prosthesis that can "float" within a native annulus may be usable for a wider variety of patient anatomies, as one or a fewer number of radial sizes of the frames can be used to fit a greater number of patients. In such embodiments, because the anchors are configured to extend further from the frame, these prostheses are still able to securely grasp native tissue as the anchors can expand to different diameters depending on how they are constrained with a body cavity. In the context of a replacement heart valve, the frame (and the associated valve body) may have the same size across multiple patient sizes, and the anchors can either be configured to expand to different diameters, or different anchor arrangements may be used for different frames.

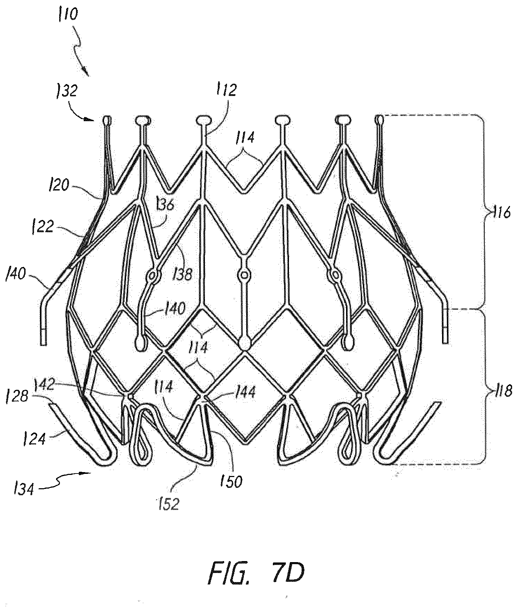

With reference to Figures 7A-D, an embodiment of a prosthesis 110 is shown. The illustrated prosthesis 110 includes a frame 120 that may be self-expanding or balloon expandable. The frame 120 (as best seen in Figure 7D) can include a proximal end 132, a distal end 134 and proximal 122 and distal 124 anchors. The anchors can allow the frame to engage a native valve annulus or other tissue to be implanted at a target location. The prosthesis 110 can include one or more of a valve 160, an outer skirt 130, a valve skirt 170 and a support band 180. The valve 160 can be designed to replace a damaged or diseased native heart valve such as a mitral valve; though it will be understood that a replacement valve is not required as part of the prosthesis.

The prosthesis can be a replacement heart valve similar to that and including features similar to those disclosed in U.S. Provisional Appl. No. 61/782,707, filed March 14, 2013, U.S. Patent No. 8,403,983 and U.S. Publication Nos. 2010/0298931, 2011/0313515 and 2012/0078353.

The frame 120 can be made of many different materials, but is preferably made from metal. In some embodiments, the frame 120 can be made from a shape memory material, such as nitinol. A wire frame or a metal tube can be used to make the frame. The wire frame of a metal tube can be cut or etched to remove all but the desired metal skeleton. In some embodiments a metal tube is laser cut in a repeating pattern to form the frame. The flat pattern can be cut from a metal tube and then the tube can be bent and expanded to the shape shown in Figures 7A-D. The frame 120 can further be expanded and/or compressed and/or otherwise worked to have the desired shape or shapes, such as for introduction and implantation.

As shown, the frame when in an expanded configuration, such as in a fully expanded configuration, has a bulbous or slightly bulbous shape, with a middle portion being larger than the proximal 132 and distal 134 ends. In some embodiments, the inside diameter of the both ends can be the same, or it can be bigger on one end than the other, while still having a middle portion larger than both the proximal and distal ends. In some embodiments, the effective diameter of the distal frame end is smaller than the effective diameter of the middle portion. The bulbous shape of the frame can advantageously allow the frame to engage a native valve annulus or other body cavity, while spacing the inlet and outlet from the heart or vessel wall. This can help reduce undesired contact between the prosthesis and the heart or vessel, such as the ventricular wall of the heart. In other embodiments, the frame may not have a bulbous portion, and can have substantially the same outer dimension along its entire length, or it may have one end larger than the other end. The prosthesis 110 and frame 120 may be similar to the replacement heart valves and associated frames, and may incorporate and/or interchange features disclosed in U.S. Provisional Appl. No. 61/782,707, U.S. Patent No. 8,403,983 and U.S. Publication Nos. 2010/0298931 and 2011/0313515.

A number of struts collectively make up the frame 120. Figures 7A-D illustrate the frame in an expanded configuration with a number of longitudinal struts 112 and undulating struts 114, with cells defined by the open spaces between the struts. The longitudinal struts may be arranged so that they are parallel or generally or substantially parallel to a longitudinal axis of the frame. The longitudinal axis of the frame may be defined as the central axis that extends through the center of the frame between the proximal 132 and distal 134 ends. Any number of configurations of struts can be used, such as the rings of undulating struts shown forming chevrons and diamonds, but also ovals, curves, and various other shapes. The illustrated embodiment includes two rings, or rows of chevrons shown in portion 116 and two rows of diamond-shaped cells shown in portion 118. The two rows of diamonds are partially obscured by the outer skirt 130.

The frame 120 has a non-foreshortening portion 116 and a foreshortening portion 118. These portions can be defined by the frame 120 and the positioning of various types of struts along the frame 120. In the figures it can be seen that the longitudinal struts 112 span the length of the non-foreshortening portion 116, while undulating struts 114 form the foreshortening portion 118. When the frame is radially collapsed or compacted, the struts 114 become more parallel with respect to the longitudinal axis of the frame, causing an outer diameter of the frame to decrease and the longitudinal length of the frame to increase in the foreshortening portion 118. As the frame moves from a compacted position to an expanded position, the longitudinal length of the frame can decrease in the foreshortening portion 118. But, the frame length does not substantially change length in the non-foreshortening portion 116.

Foreshortening of the frame 120 can be used to engage and secure the prosthesis to intralumenal tissue in a body cavity, for example tissue at or adjacent a native valve, such as a native valve annulus and/or leaflets. Opposing anchors 122, 124 can be constructed on the frame 120 so that portions of the anchors, such as tips or ends 126, 128, move closer together as the frame foreshortens. As one example, this can allow the anchors 122, 124 to grasp tissue on opposite sides of the native mitral annulus to thereby secure the prosthesis at the mitral valve.

The anchors 122, 124 and anchor tips 126, 128 can be located anywhere along the frame 120 just so long as at least one of the anchors is either connected to the foreshortening portion 118 or the foreshortening portion is positioned between the anchors so that a portion of the anchors will be move closer together with expansion of the frame. As shown, the anchors 124 are connected to the foreshortening portion 118. The foreshortening portion can also be positioned anywhere along the frame, though it is shown towards the distal end 134. In some embodiments, both of the anchor tips 126, 128 are located in the foreshortening portion 118. In some embodiments, the foreshortening portion 118 may extend the entire length of the frame, such that there is no non-foreshortening portion 116.

Preferably, each of the anchors 122, 124 is positioned or extends generally radially outwardly from the frame 120 so that the anchor tips 126, 128 are generally spaced away or radially outward from the rest of the frame 120. For example, the anchor tips may be located radially outward from the middle portion of the frame, with the tips 126 and 128 being axially spaced from one another. In some embodiments, all or part of the structure connected to the anchor tip and extending radially from the frame, including one or more rings and/or struts, can be considered part of the anchor. The anchors can include a base located on the anchor on a side opposite the tip. The base can be for example where the anchor begins to extend from or away from the frame 120.

For example, proximal anchors 122 are shown having first 136 and second 138 struts forming a chevron and connected to longitudinal struts 112 at a base of the anchor. The first and second struts of the anchor 122 are bent at the base so that the anchor 122 extends radially outwardly from the frame as it extends generally distally towards the tip 126. The first and second struts can be connected to each other at a radially outward location to form an outwardly extending loop, and in some embodiments, the first and second struts can be joined at a third strut 140 that continues to extend outwardly and/or generally distally and is then bent such that the tip points distally and extends in a manner generally parallel with the longitudinal axis of the prosthesis. The anchor also includes an eyelet 146. As illustrated, the eyelet is located along the third strut 140, though the eyelet can be positioned in other locations along the anchor 122, such as at the distal end. The tips 126 of the proximal anchors may extend distally and be parallel or substantially parallel with the longitudinal axis of the frame, or the tips 126 may extend generally distally but still radially outwardly inclined or at an acute angle relative to the longitudinal axis of the frame.

As another example, the distal anchors 124 are shown having looped anchors. Each looped anchor has a first base 142 and a second base 144 connected to the frame, wherein the first and second bases are at opposite corners of the same cell. Alternatively, the first and second bases may be located at the distal most corners of adjacent cells. The distal anchors 124 extends generally distally from the frame at the first base 142 but then is bent back around and begins to extend outwardly from the frame in a generally proximal direction. The distal anchor 124 then repeats this configuration in reverse towards the second base 144 such that the two sides of the looped anchor are mirror images of one another. It will be understood that the looped anchor can have other configurations and that it may not be symmetrical.

As illustrated in Figures 7A-D, the tips 128 of the distal anchors are circumferentially aligned with the tips 126 of the proximal anchors, though in other embodiments, the tips 128 of the distal anchors may be circumferentially staggered between the tips 126 of the proximal anchors. In the embodiment of Figures 7A-D, adjacent distal anchors 126 are spaced apart by one cell, though in other embodiments, adjacent distal anchors may be provided on adjacent cells. Thus, for example, instead of having six distal anchors and twelve proximal anchors as shown in Figures 7A-D, there may be a 1:1 correspondence between proximal and distal anchors.

The distal anchors 124 can be positioned to be not as far radially outward as the proximal anchors, and the tips 128 may be positioned radially inward of the tips 126. As described further below, such a configuration may be advantageous in positioning and securing the prosthesis in a mitral valve or other body location. As shown, the distal anchors 124 may comprise loops as described above, having a curved or arcuate atraumatic tip to minimize damage to body tissue.

The illustrated looped distal anchor is made up of the following segments. The first segment 150 extends generally longitudinally with the frame, extending distally or generally distally (e.g., slightly radially inward) with the frame. The strut is then bent back around to point in generally the opposite direction at the second segment 152. The second segment 152 ends in the rounded tip 128 and then the anchor strut repeats to form the mirror image. After the second segment 152 bends back around to point in generally the opposite direction, in the embodiment illustrated the second segment may first extend radially outward at an acute angle relative to the longitudinal axis before bending into a portion that extends parallel or substantially parallel to the longitudinal axis. The paired second segments 152 may extend parallel or generally parallel with one another at least near the tip, though they may also move slightly towards or away from each other in some embodiments. The distal anchors 124 can positioned outward from the frame and yet inward from the position of the proximal anchors 122.

It will be understood that the anchors can have various other configurations. In some embodiments, each of the anchors can extend radially outwardly from the frame at an anchor base and terminate at an anchor tip. The anchors can be connected to the frame at one of many different locations including apices, junctions, other parts of struts, etc. The anchors can comprise first, second, third, or more spaced apart bending stages along the length of each anchor. The anchors can also extend either distally or proximally before and/or after one or more of the bending stages. A portion of the anchor may extend with the frame before or after any bending stages.

In the illustrated embodiment there are twelve proximal anchors 122 and six distal anchors 124. It will be understood that other numbers and groupings of anchors can be used. For example, in some embodiments with twelve distal anchors, two anchors can share the first segment 150 where the anchor base 142, 144 is connected to the frame. In some embodiments there may be twelve anchors on one side and twelve on the other. In addition, the distal and proximal anchors may be aligned so the tips point generally towards each other, or they may be spaced so that the tips point between two tips on the opposite side.

The anchor tips 126 and 128 as described above advantageously provide atraumatic surfaces that may be used to grasp intralumenal tissue without causing unnecessary or undesired trauma to tissue. For example, the proximal anchors tips 126 and distal anchor tips 128 may form flat, substantially flat, curved or other non-sharp surfaces to allow the tips to engage and/or grasp tissue, without necessarily piercing or puncturing through tissue. A looped end or looped anchor may assist the frame in not getting caught up on structures at or near the treatment location. For example, each loop can be configured so that when the frame is deployed in-situ and expands, the movement of each loop from a delivered position to a deployed position can avoids getting caught on the papillary muscles.

The prosthesis 110 may include a valve 160. The valve 160 can be a replacement heart valve which includes a plurality of valve leaflets. The plurality of valve leaflets can function in a manner similar to the natural mitral valve, or to other valves in the vascular system. The plurality of valve leaflets can open in a first position and then engage one another to close the valve in a second position. The plurality of valve leaflets can be made to function as a one way valve such that flow in one direction opens the valve and flow in a second direction opposite the first direction closes the valve. The replacement heart valve 160 can be constructed so as to open naturally with the beating of the heart. For example, the plurality of valve leaflets can open during diastole and close during systole.

In some embodiments, the leaflets can be coupled to a valve skirt 170. For example, Figures 7A-C show a seam 162 where the proximal ends of the leaflets can be connected to the valve skirt 170.

The valve skirt 170 can be used to at least partially control how fluid flows through and/or around the valve 160. The valve skirt 170 can surround at least a portion of the valve and be connected to the valve leaflets 162. In some embodiments, the valve skirt 170 can form an inner wall connected to and positioned within the frame 120. The valve skirt 170 can also be made to move with the foreshortening portion 118 of the frame 120.

The valve skirt 170 can extend the length of the frame 120 or it can extend along only part of the length of the frame 120. In some embodiments, the ends of the heart valve 160 can coincide with ends of the valve skirt 170. In addition, one or more of the ends of the frame 120 can coincide with the ends of the valve skirt 170. In the illustrated embodiment, the proximal end of the valve skirt 170 is positioned proximally from the proximal end of the heart valve 160. The valve skirt 170 can not only extend to the distal end of the frame 120 but can also extend to the outside of the frame and is shown attached to and extending the tip 128 of each distal anchor 124. As shown, the skirt 170 is sewn to each distal anchor.

Other shapes and configurations can also be used for the valve 160 and valve skirt 170. In some embodiments, the valve skirt 170 may extend along the length of the leaflets 162, but is not connected to them. In the illustrated embodiments, the valve skirt 170 is attached to the frame 120 and the leaflets 162 are attached to the valve skirt 170.

The valve skirt 170 can be constructed in multiple different ways. The valve skirt 170 can be made of knit polyester or another stretchable or flexible fabric. In some embodiments, the valve skirt 170 is made from a material that is more flexible than the valve leaflet material. The distal and/or proximal end of the skirt 170 can be straight, curved, or have any other desired configuration. For example, the valve skirt 170 is shown with straight ends. In other embodiments the skirt distal end can be patterned to generally correspond to the undulations at the distal end 134 of the frame 120. Similarly, the proximal ends may also correspond in shape. The valve skirt 170 can be formed of one piece (according to the invention) or multiple pieces (not according to the invention). For example, the valve skirt 170 attached to the valve 160 can be one piece and then each distal anchor can be covered by a separate piece of material of the valve skirt 170. It is to be understood that other configurations of the valve skirt 170 can also be employed. For example, the anchors may remain uncovered, or only a portion may be covered.

In another embodiment of the valve skirt 170 the end can extend past the frame and can be wrapped around it. Thus, the valve skirt 170 can extend from the inside of the frame 120 to the outside of the frame. The skirt can extend completely around the frame for 1/4, 1/3, 1/2, or more of the length of the distal anchors. The skirt can also cover the distal anchors 124. The skirt can be a one piece skirt, but it will be understood that the skirt can be made of multiple pieces.

The valve skirt 170, and particularly portions that cover the distal anchors 124, can beneficially be used to help prevent leakage of blood flow around the heart valve. In addition, the skirt can encourage tissue in-growth between the skirt and the natural tissue. This may further help to prevent leakage of blood flow around the heart valve.

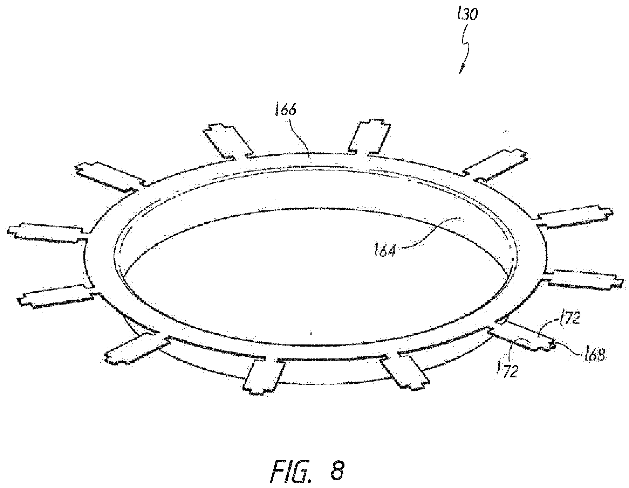

Looking to Figure 8, an outer skirt or apron 130 is shown that may also form part of the prosthesis 110. Figures 7A-C show the outer skirt 130 attached to the frame 120. The outer skirt 130 can have a portion shaped to correspond generally with the shape of an outer portion of the frame 120. For example and in accordance with the invention, a first portion 164 of the outer skirt 130 can have a cylindrical or generally cylindrical shape with an inner diameter that substantially corresponds in size to, or in embodiments not according to the invention, may be larger or slightly larger than, an outer diameter of the frame 120. In some embodiments, the first portion 164 surrounds the bulbous region of the frame and may be located surrounding the largest outer diameter of the frame 120. The outer skirt 130 can have a second portion 166 with an annular shape that extends away from the first portion 164 to an outer border with a diameter larger than the diameter of the first portion. As illustrated in Figure 8, the second portion 166 is shown flaring outward from the first portion 164 and extending generally perpendicularly from the first portion 164. Thus, the illustrated second portion forms an annular ring comprising a proximal edge and a distal edge, wherein a diameter of the proximal edge is larger than a diameter of the distal edge.

The outer skirt 130 can attach to the frame, and more preferably attach to the anchors, in one of many different ways. The outer skirt 130 can be sewn to the frame and/or valve skirt. The outer skirt 130 can also be wrapped around a portion of the frame and then sewn to itself. In the embodiments illustrated in Figures 7A-7C and 8, the second portion 166 can be attached to the proximal anchors. For example, a plurality of circumferentially spaced tabs 168 extending radially outward from the proximal edge of the second portion 166 can be used to attach the outer skirt 130 to the proximal anchors. Wings 172 on either side of the tab 168 can be wrapped around a proximal anchor and connected to each other and/or to the proximal anchor 122 to form a sleeve. The tabs 168 themselves may also form sleeves that are configured to surround at least a portion of the proximal anchors. In some embodiments, the proximal anchors 122 can include eyelets 146 that may be used to secure the skirt to the anchor. The one or both wings 172, or other parts of the tab 168, can be attached to the eyelet 146, for example by stitching the tab to the eyelet.

As shown, the eyelet 146 is spaced proximally from the end of the anchor. In other embodiments, the eyelet can be at the distal end of the anchor 122. In some embodiments, the proximal anchors can be looped anchors or have a looped end. A small tab can be passed through the looped anchor or looped end and connected to the skirt to form a loop on the skirt. Further, the outer skirt 130 may attach directly to the eyelets 146 without the need for tabs 168.

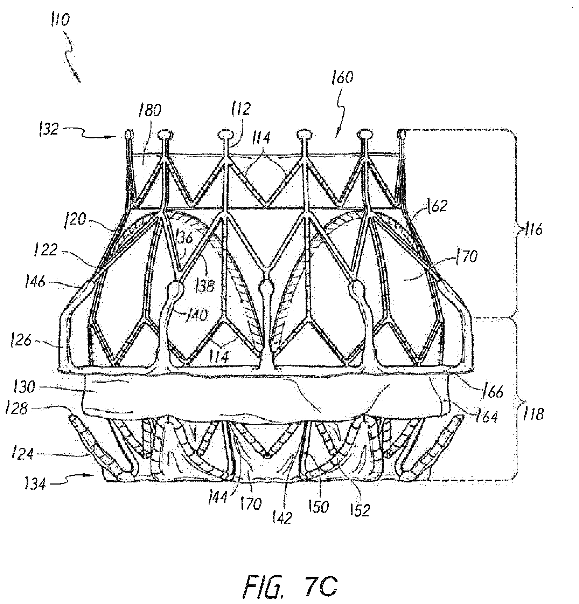

In the embodiment illustrated in Figures 7A-7C, the outer skirt 130 is only attached to the frame via the proximal anchors, and the first portion 166 remains unattached to any portion of the frame or any anchors. Thus, as illustrated in Figure 7C, the first portion 166 when attached to the frame extends distally from the proximal anchors 122 and terminates in a free distal edge. In other embodiments, the first portion 166 may also be attached to portions of the frame and/or the distal anchors. Because of the bulbous shape of the frame, the free distal edge may be spaced radially outward from the frame when the frame is in an expanded configuration.

In some embodiments, the outer skirt can attach to the frame at a distal end of the skirt, or at some other location and then curve up and out towards the proximal anchors. Thus, the outer skirt may not have a distinct first portion and second portion. In still other embodiments, the outer skirt may extend along a substantial portion of the frame. The outer skirt may be attached to the distal ends of the proximal anchors and extend to the base of the anchor and then extend along the frame to a location parallel with the ends of the proximal anchors, or even more distal still, such as to the base of the distal anchors 124.

In some embodiments, the outer skirt 130 can be part of, or connected to, the valve skirt 170, such as being connected to the valve skirt 170 at or near the distal end 134 of the frame.

The outer skirt 130 can be constructed in multiple different ways and may be made of similar material to the valve skirt 170. The outer skirt 130 can be made of a layer of resilient material, such as knit polyester or another stretchable or flexible fabric. In some embodiments, the outer skirt 130 is made from a material that is more flexible than the valve leaflet material. The distal and/or proximal end of the outer skirt 130 can be straight, curved, or have any other desired configuration. The outer skirt 130 can be formed of one piece or multiple pieces. For example, the outer skirt 130 attached to the frame 120 can be one piece and then each proximal anchor 122 can be covered by a separate piece of material of the outer skirt 130. It is to be understood that other configurations of the outer skirt 130 can also be employed. For example, the anchors may remain uncovered, or only a portion may be covered.

The prosthesis 110 can also include a support band 180 as is shown in Figures 7A-C. The support band 180 may be placed or positioned around or within the frame 120 at the proximal end 132. The support band 180 can be used to reinforce and/or constrain the frame 120. The support band 180 can help to control the expansion of the frame 120 from the compacted to the expanded state. The support band 180 can also be used to reduce the amount of motion that occurs at the proximal end 132 after the prosthesis 110 has been implanted at the mitral heart valve or other location.

In some embodiments, the support band 180 may comprise a polyester fabric band. The support band 180 may comprise a no-stretch or limited stretch material. Preferably the support band 180 is not made of an elastic material or a material known to have high elasticity. In some embodiments, the support band 180 is made from a material that is less flexible than the valve skirt material and/or the valve leaflet material. The distal and proximal ends of the support band 180 can be straight, curved, undulating with the undulations of frame, or any other desired configuration.

The support band 180 can be connected to the valve frame with a plurality of stitches, loops, knots, staples, or other types of connections. In some embodiments, the frame 120 can be sandwiched between two sides or layers of the support band 180. Preferably, the support band 180 is a single layer positioned within and attached to the frame 120 with a plurality of stitches around one or more of the longitudinal and/or undulating struts. In some embodiments, the support band 180 can be attached to the proximal end of the valve skirt 140.

The outer skirt 130 can beneficially prevent axial flow of fluid around an exterior of the prosthesis. For example, with the outer skirt 130 be positioned annularly around an exterior of the expandable frame and secured to at least some of the plurality of proximal anchors, the outer skirt creates an axial barrier to fluid flow exterior to the frame when deployed within a body cavity. In addition, the skirt can encourage tissue in-growth between the skirt and the natural tissue. This may further help to prevent leakage of blood flow around the heart valve.

In one embodiment, the outer skirt 130 can be used to help prevent leakage of blood flow around a heart valve, such as a mitral valve, when the prosthesis is placed in a native heart valve. For example, the outer skirt 130 can engage an atrial side of the mitral valve. The proximal anchors can also engage the mitral valve forcing the outer skirt 130 into close contact with the valve to block flow from passing through the mitral valve from outside of the frame.

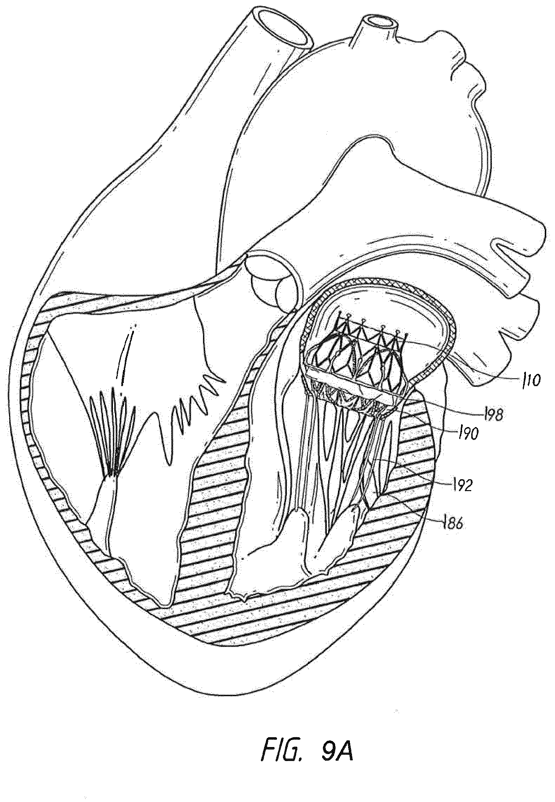

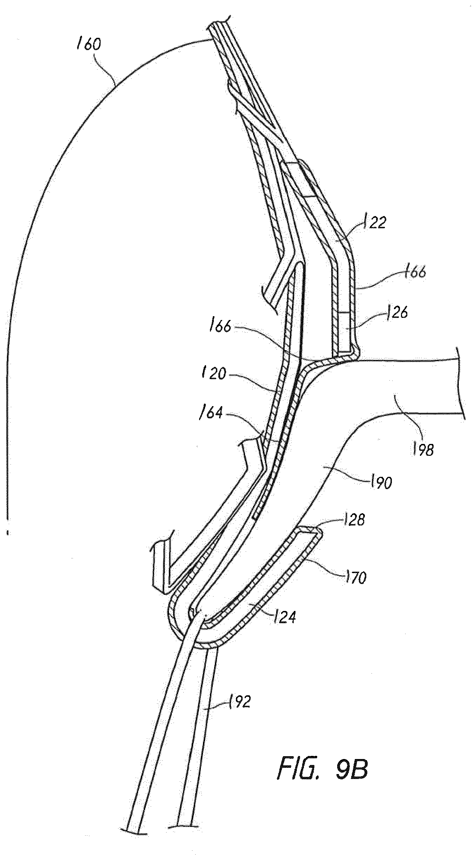

In preferred embodiments, the prostheses 110 in the form of a replacement heart such as described above may be deployed into a heart valve annulus. The prosthesis 110 may be delivered into the mitral valve in a radially compacted or collapsed configuration and positioned when compacted so that the anchor tips 126, 128 of the opposing anchors 122, 124 are disposed on opposite sides of the native annulus 198 as shown in Figures 9A and 9B. As the replacement heart valve 110 is expanded, the opposing anchors are drawn closer together and may grasp tissue on opposite sides of the native annulus 198 and securely hold the replacement heart valve 110 in position. As such, the replacement heart valve 110 can be held securely in position without requiring a substantial radial force against the native annulus. Because the anchor tips are preferably atraumatic, the grasping or engaging of tissue by the prosthesis minimizes damage to the native tissue. The foreshortening portion 118 can be used to move the anchor tips 126, 128 closer together as the replacement heart valve 110 moves to the expanded position to thereby engage the native valve annulus. The prosthesis can be deployed into a heart valve or otherwise deployed in manners similar to those described with respect to a replacement heart valve in U.S. Publication No. 2010/0298931 and 2012/0078353.

Figures 9A and 9B show a schematic representation of the replacement heart valve 110 installed in a human heart 184. The heart is shown in cross-section, and represents typical anatomy, including a left atrium 178 and left ventricle 186. The left atrium 178 and left ventricle 186 communicate with one another through a mitral annulus 198. Also shown schematically is a native anterior mitral leaflet 190 having chordae tendineae 192 that connect a downstream end of the anterior mitral leaflet 190 to the left ventricle 186.

An exemplary method of delivering a replacement valve to a native mitral valve and atraumatically securing the replacement valve relative to the native mitral valve annulus 198 is described as follows. The replacement valve can be mounted on a delivery device and delivered to the native mitral valve annulus while the replacement valve is in a radially compacted state. The replacement valve may be positioned so that the ends or tips of the distal anchors are on a ventricular side of the native leaflets 190 beyond a location where chordae tendineae 192 connect to free ends of the native leaflets. At least a portion of the replacement valve can be released from the delivery device to thereby expand the distal anchors radially outwardly. At this time the distal anchors may extend between at least some of the chordae. The distal anchors (along with the frame) can be moved toward the ventricular side of the native valve annulus with the distal anchors extending between at least some of the chordae tendineae to provide tension on the chordae tendineae. With tension provided on the chordae tendineae, the replacement valve can be further released from the delivery device to thereby expand the proximal anchors radially outwardly. The proximal anchors upon further release of the replacement valve from the delivery device can move into engagement with tissue on an atrial side of the native valve annulus, such as with the atrial side of the native valve annulus.

The method just described may utilize any of the prostheses herein described, including any of the prostheses described in the patents and applications incorporated by reference herein. In one embodiment, a prosthesis where the ends of the distal anchors are not positioned as far out radially as the ends of the proximal anchors when the frame is expanded can beneficially be used in this method. Thus, the distal anchors may have a suitable length for extending between and providing tension on the chordae tendineae, but need not and may in some embodiments not engage tissue with the tips 128. Thus, in some embodiments some or all of the distal anchors remain spaced from tissue on the ventricular side of the native valve annulus after delivery and expansion. The interaction between the distal anchors and the chordae tendineae may therefore be sufficient to secure the distal end of the prosthesis, while the engagement of the proximal anchors with tissue on the atrial side of the native valve annulus will help further secure and orient the prosthesis

As illustrated in Figures 9A and 9B, the distal anchors may comprise loops, such as any of the looped structures previously described or described in the patents and applications incorporated by reference herein. The proximal and/or distal anchors may also be covered with a resilient material such as described above for the outer skirt 130 and valve skirt 170 that promotes tissue growth with adjacent body tissue. Such material may also be useful to prevent paravalvular leakage. The atraumatic distal anchors may advantageously prevent snagging of the prosthesis on internal structures, such as the papillary muscles.

When the prosthesis is in an expanded configuration within the native mitral heart valve, the engagement of the proximal anchors 122 with tissue on the atrial side of the native mitral valve causes at least the second portion 166 of the outer skirt 130 to also engage the tissue on the atrial side of the native mitral valve. The first portion 164 of the outer skirt extends distally from the proximal anchors toward the ventricle. Because the diameter of the first portion 164 is close or the same in dimension as the frame, at least at the proximal edge of the first portion 164, the outer skirt form a barrier to blood flow around the outside or external to the frame. The outer skirt 130 can be forced against the outside of the frame 120 by the native leaflets. Where the native leaflets do not force the outer skirt 130 against the frame, or where the contact is not as strong, the outer skirt 130 is still present to block, or impede blood flow. It will be understood that having multiple contact points between the native valve and the outer skirt can allow the outer skirt to securely cover areas where there are fewer contacts between the two. As described above, the outer skirt may also promote tissue growth with tissue that it contacts.

## Claims

1. A prosthesis (10; 110) configured to grasp intralumenal tissue when deployed within a body cavity and prevent axial flow of fluid around an exterior of the prosthesis, the prosthesis comprising:
an expandable frame (20; 120) comprising a proximal end (32; 132) and a distal end (34; 134) and a longitudinal axis extending therethrough, the frame configured to radially expand and contract for deployment within the body cavity;
a plurality of proximal anchors (22; 122) **characterised in that** the plurality of anchors are each connected to the frame so that when the frame is in an expanded configuration an end of each proximal anchor is positioned radially outward from the frame and extends distally; and wherein the prosthesis also comprises
a one-piece skirt (30; 130) annularly positioned around an exterior of the expandable frame to create an axial barrier to fluid flow exterior to the frame when deployed within the body cavity, wherein the skirt includes a first portion (64; 164) that has a generally cylindrical shape with an inner diameter that substantially corresponds to an outer diameter of the frame and a second portion (66; 166) with an annular shape that extends away from the first portion to an outer border with a diameter larger than the diameter of the first portion, wherein the outer border of the second portion is attached to the frame at the tips (26; 126) of the proximal anchors.

2. The prosthesis of Claim 1, wherein at least some of the plurality of proximal anchors comprises an eyelet (46; 146), wherein the skirt is attached to the eyelets.

3. The prosthesis of any one of Claims 1-2, wherein the skirt comprises a plurality of wings (172) or sleeves that surround at least some of the proximal anchors.

4. The prosthesis of any one of Claims 1-3, wherein at least some of the plurality of proximal anchors comprise looped ends, and the skirt is attached to the looped ends.

5. The prosthesis of Claim 1, wherein the first portion of the skirt extends distally from the second portion and terminates in a distal edge.

6. The prosthesis of any one of Claims 1-5, comprising a plurality of distal anchors (24; 124) each connected to the frame so that when the frame is in an expanded configuration an end of each distal anchor is positioned radially outward from the frame and extends proximally, wherein the ends of the distal anchors are axially spaced from the ends of the proximal anchors when the frame is in an expanded configuration.

7. The prosthesis of Claim 6, further comprising a second skirt (70; 170) covering distal anchors to facilitate in-growth of adjacent tissue when the prosthesis is deployed within a body cavity.

8. The prosthesis of any one of Claims 1-7, further comprising a valve body (60; 160) attached to the frame.

9. The prosthesis of any one of Claims 1-8, wherein when the frame (120) is in an expanded configuration, the frame has a larger cross-sectional dimension in a middle portion of the frame and a smaller cross-sectional dimension in a proximal portion and a distal portion of the frame, and wherein the ends of the anchors are positioned radially outward from the middle portion of the frame.

10. The prosthesis of Claim 9, wherein the proximal end (132) and the distal end (134) have substantially the same cross-sectional dimension.

11. The prosthesis of Claim 1, wherein the second portion of the skirt extends perpendicularly from the first portion of the skirt.

12. A replacement heart valve suitable for use for securement to a native mitral valve annulus, comprising the prosthesis of any one of Claims 1-11.

## Patentansprüche

1. Prothese (10; 110), die dazu ausgelegt ist, Intraluminalgewebe zu ergreifen, wenn sie in einem Körperhohlraum ausgebracht ist, und eine Axialströmung von Fluid um ein Äußeres der Prothese herum zu verhindern, wobei die Prothese Folgendes umfasst:
einen expandierbaren Rahmen (20; 120), der ein proximales Ende (32; 132) und ein distales Ende (34; 134) und eine sich dort hindurch erstreckende Längsachse umfasst, wobei der Rahmen dazu ausgelegt ist, sich zum Ausbringen in dem Körperhohlraum radial zu expandieren und zusammenzuziehen,
ene Vielzahl von proximalen Ankern (22; 122),
**dadurch gekennzeichnet, dass** die Vielzahl von Ankern jeweils mit dem Rahmen verbunden sind, so dass ein Ende jedes proximalen Ankers radial außerhalb des Rahmens positioniert ist und sich distal erstreckt, wenn der Rahmen in einer expandierten Konfiguration ist, und wobei die Prothese auch einen einteiligen Mantel (30; 130) umfasst, der ringförmig um ein Äußeres des expandierbaren Rahmens positioniert ist, um eine axiale Sperre gegen Fluidströmung außerhalb des Rahmens zu erzeugen, wenn sie in dem Körperhohlraum ausgebracht ist, wobei der Mantel einen ersten Abschnitt (64; 164), der eine allgemein zylindrische Form mit einem Innendurchmesser hat, der einem Außendurchmesser des Rahmens im Wesentlichen entspricht, und einen zweiten Abschnitt (66; 166) mit einer ringförmigen Form aufweist, der sich von dem ersten Abschnitt weg zu einem äußeren Rand mit einem Durchmesser erstreckt, der größer als der Durchmesser des ersten Abschnitts ist, wobei der äußere Rand des zweiten Abschnitts an den Spitzen (26; 126) der proximalen Anker an dem Rahmen angebracht ist.

2. Prothese nach Anspruch 1, wobei mindestens einige der Vielzahl von proximalen Ankern eines Öse (46; 146) umfassen, wobei der Mantel an den Ösen angebracht ist.

3. Prothese nach einem der Ansprüche 1 - 2, wobei der Mantel eine Vielzahl von Flügeln (172) oder Hülsen umfasst, die mindestens einige der proximalen Anker umgeben.

4. Prothese nach einem der Ansprüche 1 - 3, wobei mindestens einige der Vielzahl von proximalen Ankern Schlaufenenden umfassen und der Mantel an den Schlaufenenden angebracht ist.

5. Prothese nach Anspruch 1, wobei sich der erste Abschnitt des Mantels distal von dem zweiten Abschnitt erstreckt und in einem distalen Rand endet.

6. Prothese nach einem der Ansprüche 1 - 5, umfassend eine Vielzahl von distalen Ankern (24; 124), die jeweils mit dem Rahmen verbunden sind, so dass ein Ende jedes distalen Ankers radial außerhalb von dem Rahmen positioniert ist und sich proximal erstreckt, wenn der Rahmen in einer expandierten Konfiguration ist, wobei die Enden der distalen Anker von den Enden der proximalen Anker axial beabstandet sind, wenn der Rahmen in einer expandierten Konfiguration ist.

7. Prothese nach Anspruch 6, ferner umfassend einen zweiten Mantel (70; 170), der distale Anker abdeckt, um das Hineinwachsen von benachbartem Gewebe zu ermöglichen, wenn die Prothese in einem Körperhohlraum ausgebracht ist.

8. Prothese nach einem der Ansprüche 1 - 7, ferner umfassend einen Klappenkörper (60; 160), der an dem Rahmen angebracht ist.

9. Prothese nach einem der Ansprüche 1 - 8, wobei der Rahmen in einem mittleren Abschnitt des Rahmens eine größere Querschnittsabmessung hat und in einem proximalen Abschnitt und einem distalen Abschnitt des Rahmens eine kleinere Querschnittsabmessung hat, wenn der Rahmen (20) in einer expandierten Konfiguration ist, und wobei die Enden der Anker radial außerhalb von dem mittleren Abschnitt des Rahmens positioniert sind.

10. Prothese nach Anspruch 9, wobei das proximale Ende (132) und das distale Ende (134) im Wesentlichen dieselbe Querschnittsabmessung haben.

11. Prothese nach Anspruch 1, wobei sich der zweite Abschnitt des Mantels senkrecht von dem ersten Abschnitt des Mantels erstreckt.

12. Ersatzherzklappe, die sich für die Verwendung zum Befestigen an einem nativen Mitralklappenanulus eignet, umfassend die Prothese nach einem der Ansprüche 1 - 11.

## Revendications

1. Prothèse (10 ; 110) configurée pour saisir un tissu intraluminal lorsqu'elle est déployée à l'intérieur d'une cavité corporelle et empêcher un écoulement axial de fluide autour d'un extérieur de la prothèse, la prothèse comprenant :
un cadre déployable (20 ; 120) comprenant une extrémité proximale (32 ; 132) et une extrémité distale (34 ; 134) et un axe longitudinal s'étendant à travers celles-ci, le cadre étant configuré pour se déployer et se contracter radialement pour un déploiement à l'intérieur de la cavité corporelle ;
une pluralité d'ancrages proximaux (22 ; 122) **caractérisée en ce que** les multiples ancrages sont reliés chacun au cadre de sorte que lorsque le cadre est dans une configuration déployée une extrémité de chaque ancrage proximal soit positionnée radialement vers l'extérieur depuis le cadre et s'étende distalement ; et dans laquelle la prothèse comprend également
une jupe en une seule pièce (30 ; 130) positionnée de manière annulaire autour d'un extérieur du cadre déployable pour créer une barrière axiale à l'écoulement de fluide à l'extérieur du cadre lorsqu'il est déployé à l'intérieur de la cavité corporelle, dans lequel la jupe comprend une première partie (64 ; 164) qui a une forme généralement cylindrique avec un diamètre interne qui correspond sensiblement à un diamètre externe du cadre et une seconde partie (66 ; 166) avec une forme annulaire qui s'étend à l'opposé de la première partie jusqu'à une bordure externe avec un diamètre plus grand que le diamètre de la première partie, dans lequel la bordure externe de la seconde partie est fixée au cadre au niveau des pointes (26 ; 126) des ancrages proximaux.

2. Prothèse selon la revendication 1, dans laquelle au moins certains ancrages de la pluralité d'ancrages proximaux comprennent un œillet (46 ; 146), dans laquelle la jupe est fixée aux œillets.

3. Prothèse selon l'une quelconque des revendications 1 et 2, dans laquelle la jupe comprend une pluralité d'ailes (172) ou de manchons qui entourent au moins certains des ancrages proximaux.

4. Prothèse selon l'une quelconque des revendications 1 à 3, dans laquelle au moins certains ancrages de la pluralité d'ancrages proximaux comprennent des extrémités en boucle, et la jupe est fixée aux extrémités en boucle.

5. Prothèse selon la revendication 1, dans laquelle la première partie de la jupe s'étend distalement depuis la seconde partie et se termine par un bord distal.

6. Prothèse selon l'une quelconque des revendications 1 à 5, comprenant une pluralité d'ancrages distaux (24 ; 124) reliés chacun au cadre de sorte que lorsque le cadre est dans une configuration déployée une extrémité de chaque ancrage distal soit positionnée radialement à l'extérieur du cadre et s'étende proximalement, dans laquelle les extrémités des ancrages distaux sont espacées axialement des extrémités des ancrages proximaux lorsque le cadre est dans une configuration déployée.

7. Prothèse selon la revendication 6, comprenant en outre une seconde jupe (70 ; 170) recouvrant les ancrages distaux pour faciliter la croissance interne de tissu adjacent lorsque la prothèse est déployée à l'intérieur d'une cavité corporelle.

8. Prothèse selon l'une quelconque des revendications 1 à 7, comprenant en outre un corps de soupape (60 ; 160) fixé au cadre.

9. Prothèse selon l'une quelconque des revendications 1 à 8, dans laquelle lorsque le cadre (120) est dans une configuration déployée, le cadre a une dimension de coupe transversale plus grande dans une partie centrale du cadre et une dimension de coupe transversale plus petite dans une partie proximale et une partie distale du cadre, et dans laquelle les extrémités des ancrages sont positionnées radialement à l'extérieur de la partie centrale du cadre.

10. Prothèse selon la revendication 9, dans laquelle l'extrémité proximale (132) et l'extrémité distale (134) ont sensiblement la même dimension en coupe transversale.

11. Prothèse selon la revendication 1, dans laquelle la seconde partie de la jupe s'étend perpendiculairement à la première partie de la jupe.

12. Valve cardiaque de remplacement pouvant être utilisée pour être fixée à l'anneau d'une valve mitrale native, comprenant la prothèse selon l'une quelconque des revendications 1 à 11.
